# EUROPEAN PATENT APPLICATION

(11) **EP 4 678 654 A1**
(43) Date of publication of application: **14.01.2026**
(21) Application number: 24769588.5
(22) Date of filing: 11.03.2024
(51) Int. Cl.: C07K 14/20, C07K 19/00, A61K 38/16, G01N 33/569, C12N 1/15, C12N 1/21, C12N 5/10, C12N 15/31, C12N 15/62, C12N 15/63, C12R 1/19, C12R 1/84

(54) **CHIMERIC PROTEIN, HOST MICROBIAL CELL, PROCESS FOR OBTAINING SAID CHIMERIC PROTEIN, LEPTOSPIROSIS SEROLOGICAL DIAGNOSTIC KIT, IN VITRO METHOD FOR EARLY SEROLOGICAL DIAGNOSIS OF LEPTOSPIROSIS AND USE OF SAID CHIMERIC PROTEIN**

(30) Priority: 10.03.2023 BR 102023004571
(71) Applicant: INSTITUTO BUTANTAN, 05503-000 São Paulo (BR)
(72) Inventor: VIRGÍLIO FERNANDES, Luis Guilherme, Pirajuí, SP 16600-055 (BR); TABET OLLER DO NASCIMENTO, Ana Lúcia, 05433-001 SÃO PAULO (BR)
(74) Representative: Maiwald GmbH
(86) International application number: PCT/BR2024/050085
(87) International publication number: WO 2024/187254

(57) **Abstract**

The present invention relates to a chimeric protein composed of specific regions of proteins proven to be conserved and present on the surface of *L. interrogans* selected from the group consisting of LIC10123, LIC13059, LIC13050, LIC12615, LIC11636, LIC11352, LIC11122, LIC11089, LIC10713, and LIC13434, wherein the specific regions consist of the amino acid sequences as established by SEQ ID NO: 2, 4, 6, 7, 9, 11, 13, 14, 16, 18, 20, and 22. The present invention further relates to a host microbial cell comprising the chimeric gene consisting of the nucleotide sequence as established by SEQ ID NO: 25, the chimeric gene and an expression cassette. In addition, the present invention relates to a process for obtaining said chimeric protein and a leptospirosis serological diagnostic kit comprising the chimeric protein of the present invention. Additionally, the present invention relates to an *in vitro* method for early serological diagnosis of leptospirosis and the use of said chimeric protein for early serological diagnosis of leptospirosis by detecting IgM and/or IgG antibodies in serum samples from patients suspected of having leptospirosis.

## Description

### Field of Application:

The present invention falls within the field of peptides, more specifically in the area of investigation or analysis of materials by determining their chemical or physical properties, since it refers to a chimeric protein for early serodiagnosis of leptospirosis.

### Background of the Invention and State of the Art:

Leptospirosis, caused by pathogenic bacteria of the genus *Leptospira,* is considered the zoonosis of greatest global importance. The difficulty in making a quick and early diagnosis of the disease results in a high mortality rate, due to the delay in starting antibiotic therapy.

The test recommended by the WHO, called the Microagglutination Test (MAT), is based on detecting the agglutination of leptospires by antibodies present in the patient's serum, but it has low sensitivity in the early stages of the disease and is difficult to perform. Thus, antigens are needed that are more sensitive to detecting antibodies in the early stages of the disease.

In order to solve the existing technical problem, the present invention proposes the development of a chimeric protein based on proteins proven to be conserved and present on the surface of *L. interrogans,* in which regions of functional domains and with incidence of B lymphocyte epitopes capable of stimulating the production of antibodies were selected. The presence of portions of 10 distinct proteins increased the sensitivity of a serological diagnosis, making it possible to determine a positive diagnosis by the concomitant detection of IgM and IgG antibodies against the chimeric protein in 75% of serum samples from leptospirosis patients that were false-negative by the microagglutination test (MAT). In this sense, the chimeric protein of the present invention favors the early diagnosis of leptospirosis.

Some state-of-the-art documents describe recombinant proteins for use in early leptospirosis serodiagnosis.

Indian patent application No. IN 04438CH2015 A, published on November 14, 2019, in the name of BHARATHIDASAN UNIVERSITY, entitled: *"B and T cell specific peptides of leptospiral protein LK90 for the diagnosis leptospirosis"* describes a method for the diagnosis of leptospirosis. It describes a diagnostic method of leptospirosis-specific B- and T-cell peptides from the leptospiral protein LK90 consisting of 3 (three) peptides for the enhanced diagnosis of leptospirosis in the early stages. However, the document distances itself from the present invention, since the present invention proposes a chimeric protein composed of 10 (ten) immunogenic conserved proteins present on the surface of *L. interrogans,* which detects IgM and/or IgG type antibodies in serum samples in a rapid *point-of-care* test.

Indian patent application No. IN 01867CH2014 A, published on April 25, 2014, in the name of COORDINATOR NATIONAL HUB FOR HEALTHCARE INSTRUMENTATION DEVELOPMENT; DIRECTOR TAMIL NADU VETERINARY AND ANIMAL SCIENCES UNIVERSITY; and REGISTRAR, entitled: *"Novel multi-epitope antigen for diagnosis of leptospirosis"* describes a method for rapid detection of leptospirosis in humans, using a recombinant multi-epitope leptospiral protein designed by assembling the conserved regions of 4 (four) outer membrane antigenic proteins. However, the document differs from the present invention, since the present invention proposes a chimeric protein composed of 10 (ten) conserved immunogenic proteins present on the surface of *L. interrogans,* which detects IgM and/or IgG antibodies in serum samples.

US Patent Application No. US 2015/0004623 (A1), published on January 1, 2015, in the name of BAHAMAN ABDUL RANI and SEENICHAMY ARIVUDAINAMBI, entitled: *"Diagnostic kit for the detection of early acute leptospirosis"* describes an antigen composition comprising at least 2 immunogenic conserved proteins present on the surface of *L. interrogans,* which is for use in the diagnosis of leptospirosis in a mammal, comprising two or more antibody-reactive antigens that are associated with a carrier. However, the document differs from the present invention, since the present invention proposes a chimeric protein composed of 10 (ten) immunogenic conserved proteins present on the surface of *L. interrogans* different from those proposed in the aforementioned prior art document, which detects IgM and/or IgG type antibodies in serum samples for early diagnosis of leptospirosis.

U.S. Patent Application No. US 2012/100143 (A1), published on April 26, 2012, in the name of CHANG YUNG-FU, entitled: *"Immunogenic proteins from genome-derived outer membrane of leptospira and compositions and methods based thereon"* describes a method for diagnosing a Leptospira-related disorder in a subject, which comprises the use of a chimeric protein composed of 12 (twelve) immunogenic conserved proteins present on the surface of *L. interrogans.* However, the document differs from the present invention, since the present invention proposes a chimeric protein composed of 10 (ten) immunogenic conserved proteins present on the surface of *L. interrogans* different from those proposed in the aforementioned prior art document, which detects IgM and/or IgG type antibodies in serum samples for early diagnosis of leptospirosis.

US patent application No. US 2017/021004 (A1), published on January 26, 2017, in the name of MERIAL, INC, GENOSTAR, VETAGRO-SUP and INSTITUT PASTEUR, entitled: *"Leptospira Immunoprotective Proteins and Methods of Identification and Use Thereof" (Leptospira* immunoprotective proteins and methods of identification and use thereof) describes a method of identifying immunoprotective proteins of *Leptospira spp.,* and the use of one or more immunogenic conserved proteins present on the surface of *L. interrogans* for the detection of IgM and/or IgG antibodies in serum samples. However, the document differs from the present invention, since the present invention proposes a chimeric protein composed of 10 (ten) immunogenic conserved proteins present on the surface of *L. interrogans* different from those proposed in the aforementioned prior art document, which detects IgM and/or IgG type antibodies in serum samples for early diagnosis of leptospirosis.

Therefore, based on the aforementioned state of the art, the need for early diagnostic tests for leptospirosis is a common technical problem. the use of a recombinant protein composed of epitopes of conserved immunogenic proteins present on the surface of *L. interrogans.* interrogans. However, the development of said chimeric protein of the present invention composed of a specific combination of 10 immunogenic conserved protein epitopes present on the surface of *L. interrogans* for early diagnosis of leptospirosis is not described nor is it obvious in light of the state of the art. In this sense, the present invention is an alternative solution to an existing technical problem in the state of the art.

Advantageously, said chimeric protein of the present invention allows the serological diagnosis of leptospirosis, by means of the detection of IgM and/or IgG type antibodies in serum samples of patients with suspected leptospirosis, and said chimeric protein can be used for ELISA experiments or for immobilization in biosensors for rapid *point-of-care* tests. Both techniques are cheaper and easier to perform than the WHO reference test.

Thus, no state-of-the-art document discloses a chimeric protein composed of the exact 10 epitopes of immunogenic conserved proteins present on the surface of *L*. interrogans for early diagnosis of leptospirosis, in a biosensor platform for point-of-care testing described here. The invention facilitates rapid diagnosis that can be carried out in the field without the need for equipment.

### Summary of the Invention:

The present invention will provide significant advantages for the early diagnosis of leptospirosis.

In a first aspect, the present invention relates to a chimeric protein composed of specific regions of proteins proven to be conserved and present on the surface of *L*. interrogans. interrogans selected from the group consisting of LIC10123, LIC13059, LIC13050, LIC12615, LIC11636, LIC11352, LIC11122, LIC11089, LIC10713, and LIC13434, wherein the specific regions consist of the amino acid sequences as established by SEQ ID NO: 2, 4, 6, 7, 9, 11, 13, 14, 16, 18, 20, and 22.

In a second aspect, the present invention relates to a host cell comprising the chimeric gene consisting of the nucleotide sequence as set forth by SEQ ID NO: 25 or degenerates thereof encoding a protein consisting of the amino acid sequence as set forth by SEQ ID NO: 2, 4, 6, 7, 9, 11, 13, 14, 16, 18, 20, 22, or 24.

In a third aspect, the present invention relates to a process for obtaining the chimeric protein comprising the steps of: (a) transforming the host cell with an isopropyl β-D-1-thiogalactopyranoside (IPTG)-induced recombinant plasmid comprising the chimeric gene as set forth by SEQ ID NO: 25 or degenerates thereof that encode a protein consisting of the amino acid sequence as established by SEQ ID NO: 2, 4, 6, 7, 9, 11, 13, 14, 16, 18, 20, 22, or 24; and (b) purify the recombinant chimeric protein obtained by chromatography.

In a fourth aspect, the present invention relates to a leptospirosis serological diagnostic kit comprising the chimeric protein of the present invention.

In a fifth aspect, the present invention relates to an *in vitro* method for early serological diagnosis of leptospirosis comprising the steps of: (a) immobilizing 100 to 500 ng/per well of the chimeric protein of the present invention in a well plate for ELISA; (b) after blocking the ELISA wells with a solution containing an inert protein, adding the serum of an individual suspected of having leptospirosis, preferably at a dilution of 1:50 to 1:400, and incubating, preferably for 1 to 2 hours, at a temperature of about 37 °C; and (c) After washing away unbound antibodies, adding a solution containing anti-human IgM and anti-human IgG secondary antibodies conjugated with peroxidase, at a dilution of 1:5,000 to 1:10,000, and subsequently adding a chromogenic substrate.

In a sixth aspect, the present invention relates to the use of the chimeric protein of the present invention for the early serological diagnosis of leptospirosis by detecting IgM and/or IgG antibodies in serum samples from patients with suspected leptospirosis. It can be used for ELISA experiments or for immobilization in biosensors for rapid *point-of-care* tests.

In a seventh aspect, the present invention relates to an expression cassette comprising the nucleotide sequences encoding each peptide, operably linked to a heterologous promoter.

In an eighth aspect, the present invention relates to the chimeric gene encoding the chimeric protein of the invention consisting of the nucleotide sequence as set forth by SEQ ID NO: 25 or degenerates thereof encoding a protein consisting of the amino acid sequence as set forth by SEQ ID NO: 2, 4, 6, 7, 9, 11, 13, 14, 16, 18, 20, 22, or 24.

### Brief description of the figures:

The present invention, together with its additional advantages, can be better understood by referring to the attached images and the following description.
Figure 1 illustrates the composition of the chimeric protein.
Figure 2 graphically shows the reactivity with sera from patients diagnosed with leptospirosis, both in paired samples from the same patient in the initial phase (MAT-) and convalescent phase (MAT+), as well as sera from patients only in the MAT+ phase using the chimeric protein of the present invention, and with serum from healthy patients (normal serum). Reactive individuals have an absorbance above the dashed line (*cutoff* value).

### Detailed description of the invention:

Although the present invention may be susceptible to different embodiments, a preferred embodiment is shown in the following detailed discussion, with the understanding that the present embodiment should be considered an exemplification of the principles of the invention and is not intended to limit the present invention to what has been described in this report.

The present invention relates to a chimeric protein composed of specific regions of proteins proven to be conserved and present on the surface of *L*. interrogans. interrogans selected from the group consisting of LIC10123, LIC13059, LIC13050, LIC12615, LIC11636, LIC11352, LIC11122, LIC11089, LIC10713, and LIC13434, wherein the specific regions are as follows:
i. Region comprising the amino acid at position 132 to the amino acid at position 191 of the LIC10123 protein as established by SEQ ID NO: 1, wherein the specific region selected consists of the amino acid sequence of SEQ ID NO:2;
ii. Region comprising the amino acid at position 76 to the amino acid at position 150 of the LIC13059 protein as established by SEQ ID NO: 3, where the specific region selected consists of the amino acid sequence of SEQ ID NO:4;
iii. Region comprising the amino acid from position 101 to the amino acid from position 140 of the LIC13050 protein as set forth by SEQ ID NO: 5, and region comprising the amino acid from position 288 to the amino acid from position 324 of the LIC13050 protein as established by SEQ ID NO: 5, where the specific region selected consists of the amino acid sequence of SEQ ID NO:6 and 7, respectively;
iv. Region comprising the amino acid at position 120 to the amino acid at position 205 of the LIC12615 protein as established by SEQ ID NO: 8, wherein the specific region selected consists of the amino acid sequence of SEQ ID NO:9;
v. Region comprising amino acid position 70 to amino acid position 155 of protein LIC11636 as established by SEQ ID NO: 10, wherein the specific region selected consists of the amino acid sequence of SEQ ID NO:11;
vi. Region comprising amino acid position 108 to amino acid position 138 of the LIC11352 protein as set forth by SEQ ID NO: 12, and region comprising amino acid position 220 to amino acid position 272 of the LIC11352 protein as set forth by SEQ ID NO: 12, wherein the specific region selected consists of the amino acid sequence of SEQ ID NO: 13 and 14, respectively;
vii. Region comprising the amino acid at position 84 to the amino acid at position 155 of the LIC11122 protein as established by SEQ ID NO: 15, wherein the specific region selected consists of the amino acid sequence of SEQ ID NO:16;
viii. Region comprising amino acid position 30 to amino acid position 105 of protein LIC11089 as established by SEQ ID NO: 17, wherein the specific region selected consists of the amino acid sequence of SEQ ID NO:18;
ix. Region comprising amino acid position 161 to amino acid position 203 of protein LIC10713 as established by SEQ ID NO: 19, wherein the specific region selected consists of the amino acid sequence of SEQ ID NO:20; and
x. Region comprising amino acid position 374 to amino acid position 464 of the LIC13434 protein as set forth by SEQ ID NO:21, wherein the specific region selected consists of the amino acid sequence of SEQ ID NO: 22.

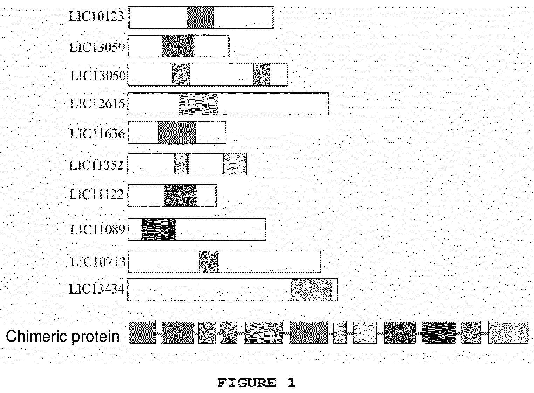

Figure 1 illustrates the regions with conserved domains and a high concentration of epitopes selected for the final sequence of the chimeric protein of the present invention.

Each region described here is connected by a glycine linker to space out each region and encourage the individual folding of each portion.

In an embodiment of the chimeric protein, this linker consists of the amino acid sequence as established by SEQ ID NO: 23.

In addition, six histidine amino acids (His6x) were included at the beginning of the recombinant chimeric protein sequence to facilitate the purification steps.

In one embodiment of the invention, the molecular weight of said chimeric protein ranges from 91.73 to 100 KDa, preferably 94.65 KDa.

The size of the final chimeric protein may vary according to the plasmid used for expression. The minimum size, considering methionine (necessarily the first amino acid), 6 histidines and the chimeric sequence, would be 91.73 kDa. The maximum size may vary depending on the additional sequences that a given plasmid may incorporate. In one embodiment, an expressed protein, considering the amino acids encoded by the plasmid in which gene was synthesized, the expected size is 94.65 kDa.

In one embodiment of the invention, said chimeric protein consists of the amino acid sequence as established by SEQ ID NO: 24.

To obtain this chimeric protein, based on its final amino acid sequence, the nucleotide sequence was generated by reverse translation for synthesis of the chimeric gene on an expression plasmid. The expression was preferably in *Escherichia coli,* but could also be in *Pichia* pastoris, mammalian cells, insect cells or *"cell-free"* protein expression kits.

Accordingly, the present invention relates to a host cell comprising the chimeric gene consisting of the nucleotide sequence as set forth by SEQ ID NO: 25 or degenerates thereof encoding a protein consisting of the amino acid sequence as set forth by SEQ ID NO: 2, 4, 6, 7, 9, 11, 13, 14, 16, 18, 20, 22, or 24.

The host cell is selected from a group consisting of *Escherichia* coli or *Pichia pastoris,* mammalian cells or insect cells, preferably *E. coli.*

In addition, the present invention relates to an expression cassette comprising the nucleotide sequences encoding each peptide, operably linked to a heterologous promoter.

Furthermore, the present invention relates to the chimeric gene encoding the chimeric protein of the present invention consisting of the nucleotide sequence as set forth by SEQ ID NO: 25 or degenerates thereof encoding a protein consisting of the amino acid sequence as set forth by SEQ ID NO: 2, 4, 6, 7, 9, 11, 13, 14, 16, 18, 20, 22, or 24.

In addition, the invention also relates to the process for obtaining the chimeric protein, which comprises the following steps:
a) Transform the host cell with a recombinant plasmid induced by isopropyl β-D-1-thiogalactopyranoside (IPTG) comprising the chimeric gene as established by SEQ ID NO: 25 or degenerates thereof encoding a protein consisting of the amino acid sequence as set forth by SEQ ID NO: 2, 4, 6, 7, 9, 11, 13, 14, 16, 18, 20, 22, or 24; and
b) Purify the recombinant chimeric protein obtained by chromatography.

In step "a", induction is carried out at a temperature ranging from 18 to 37 °C for 3 to 16 hours. In one embodiment of the invention, in step "a", induction is carried out at 18 °C for 16 hours, where the recombinant protein obtained showed greater integrity.

Also, in step "a", a concentration of 0.01 to 1 mM of IPTG is used, preferably 1 mM. Induction is carried out after the culture reaches an optical density (OD600nm) of 0.6, in Luria-Bertani (LB) medium, in a volume ranging from 200 to 800 mL.

This recombinant plasmid can be any plasmid in the state of the art that enables the expression of the recombinant protein.

In one embodiment of the invention, the host cell used is *E. coli* BL21 DE3.

In step "b", the recombinant protein is recovered from the insoluble fraction after bacterial lysis, in the form of an inclusion corpuscle. After solubilization in 8M urea solution, the protein is loaded onto a Sepharose column loaded with Nickel, after which the urea is diluted by passing through decreasing solutions of urea (6, 4, 2, and 1 M). The column is washed with increasing solutions of imidazole (10, 20, 40, and 60 mM) and then the chimeric protein is eluted with 1M imidazole solution.

In one embodiment, the recombinant chimeric protein obtained is purified by ion exchange chromatography or metal affinity chromatography, preferably by metal affinity chromatography.

In addition, the present invention relates to a leptospirosis serological diagnostic kit comprising the chimeric protein of the present invention.

In an embodiment of the present invention, said kit further comprises a veterinary and/or pharmaceutically acceptable vehicle.

In one embodiment, the final protein is dialyzed in a buffer solution, which can be 20 mM Tris-HCl (pH 8.0), 500 mM NaCl, or PBS.

In an embodiment of the present invention, said kit comprises a biosensor platform for *point-of-care* testing.

In addition, the present invention relates to an *in vitro* method for the early serological diagnosis of leptospirosis comprising the following steps:
a) Immobilize 100 to 500 ng/per well of the chimeric protein of the present invention in an ELISA well plate;
b) After blocking the ELISA wells with a solution containing an inert protein, add the serum of an individual with suspected leptospirosis at a dilution of 1:50 to 1:400, and incubate preferably for 1 to 2 hours at a temperature of around 37 °C;
c) After washing off the unbound antibodies, add a solution containing peroxidase-conjugated anti-human IgM and anti-human IgG secondary antibodies at a dilution of 1:5,000 to 1:10,000, and then add a chromogenic substrate.

The term "blocking the ELISA wells" consists of blocking the protein sites on the surface of the wells in order to avoid a subsequent nonspecific reaction. This is to ensure that the antibodies will bind specifically to the antigen and not nonspecifically to the surface of the plate. For blocking, a solution containing an inert protein such as casein (using 10% skimmed milk powder) or bovine serum albumin (1-3%) is used.

In an embodiment, the blockade is preferably performed with a solution containing casein (skimmed milk) or bovine serum albumin.

Thus, IgM and IgG antibodies resulting from infection will bind to the chimeric protein of the present invention resulting in a positive test for leptospirosis, if this is the case.

Finally, said invention also relates to the use of the chimeric protein of the present invention for the early serological diagnosis of leptospirosis by detecting IgM and/or IgG antibodies in serum samples from patients suspected of having leptospirosis, which can be used for ELISA experiments or for immobilization in biosensors for rapid *point-of-care.*

Therefore, in order to elucidate the present invention, experimental results and embodiments of the invention are presented below in order to highlight the inventive step of using the chimeric protein of the present invention.

### Embodiments of the invention

The amino acid sequences of the 10 proteins (LIC10123, LIC13059, LIC13050, LIC12615, LIC11636, LIC11352, LIC11122, LIC11089, LIC10713, and LIC13434) were selected based on previous surface proteome studies of *L. interrogans* and were subjected to bioinformatics analyses to evaluate the presence of conserved domains and B lymphocyte epitopes.

The conservation of the proteins in different species and serovars of *Leptospira* was also evaluated, showing that they are highly conserved. Regions with conserved domains and a high concentration of epitopes were selected to compose the final sequence of the chimeric protein (Figure 1). Each region was connected by a glycine linker.

Based on the final amino acid sequence of the chimeric protein, the nucleotide sequence was generated by reverse translation for the synthesis of the chimeric gene in an expression plasmid in *E. coli* induced by IPTG.

The *E. coli* BL21 DE3 strain was transformed with the recombinant plasmid and the best condition selected was induction at 18 °C for 16 hours, in which the recombinant protein (around 100 kDa) showed the greatest integrity.

Six histidine amino acids (His6x) were included at the beginning of the recombinant protein sequence, here named rQ2, to facilitate the purification steps. The recombinant chimeric protein was purified by metal affinity chromatography.

### Experimental Results

In order to demonstrate the potential of rQ2 in the serological diagnosis of leptospirosis, especially in MAT-negative samples (which are in the initial phase of the disease and are false-negative by the MAT test), an ELISA experiment was carried out.

To do this, the chimeric protein was immobilized in a 96-well plate, using 400 ng per well; after blocking, a mixture of sera from patients in the initial phase (MAT-negative) and convalescent phase (MAT-positive) was added to the wells in different dilutions (1:100 to 1:800) and then incubated for 1 hour at 37 °C. At this point, antibodies resulting from the infection bind to the chimeric antigen.

The reactivity of both IgM and IgG antibodies was detected by incubation with peroxidase-conjugated anti-human IgM and anti-human IgG secondary antibodies, after which a chromogenic substrate was used.

The results show that IgM and IgG antibodies against rQ2 were detected in both MAT-negative and positive samples, and sera from healthy patients (normal human serum) showed no reactivity with the chimeric antigen, demonstrating the specificity of the reaction.

Using the ELISA method described above, the reactivity of MAT-negative (n=36) and MAT-positive (n=178) serum samples at a dilution of 1:100 against the immobilized chimeric protein in the plate wells was evaluated. To increase the discriminatory power of the protocol, IgM and IgG antibodies were detected concomitantly. Fifty serum samples from healthy individuals proven negative for leptospirosis were also used to calculate the *cutoff* value, above which a sample is considered positive.

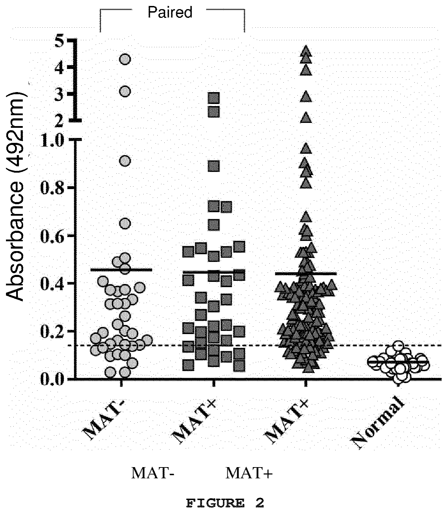

The cutoff value was calculated as the average between the absorbances obtained by ELISA for the 50 serum samples from healthy individuals plus 3 times the standard deviation between them. Based on the results, the cutoff value was 0.14. Among the 36 samples from individuals who have leptospirosis but have not yet been correctly diagnosed (false negatives), 27 (75%) presented absorbance above the *cutoff* value and were considered positive by the *in vitro* method of the present invention (Figure 2).

The average absorbance in this group was 0.45, showing a high reactivity of IgM and IgG antibodies against the chimeric antigen. In MAT-positive samples, 147 (82.5%) were above the cutoff value with an average absorbance of 0.45 (Figure 2).

Therefore, the results indicate that the chimeric protein of the present invention is more sensitive than the test recommended by the WHO (MAT) and therefore more efficient for the early diagnosis of leptospirosis.

Briefly, the invention relates to the following aspects, as defined in the following numbered items:
1. Recombinant chimeric protein comprising specific regions of proteins proven to be conserved and present on the surface of *L.* interrogans. interrogans selected from the group consisting of LIC10123, LIC13059, LIC13050, LIC12615, LIC11636, LIC11352, LIC11122, LIC11089, LIC10713, and LIC13434, where the specific regions consist of:
   i. Region comprising the amino acid at position 132 to the amino acid at position 191 of the LIC10123 protein as established by SEQ ID NO: 1, wherein the specific region selected consists of the amino acid sequence of SEQ ID NO:2;
   ii. Region comprising amino acid position 76 to amino acid position 150 of the LIC13059 protein as set forth by SEQ ID NO: 3, wherein the specific region selected consists of the amino acid sequence of SEQ ID NO: 4;
   iii. Region comprising the amino acid from position 101 to the amino acid from position 140 of the LIC13050 protein as set forth by SEQ ID NO: 5, and region comprising the amino acid from position 288 to the amino acid from position 324 of the LIC13050 protein as established by SEQ ID NO: 5, where the specific region selected consists of the amino acid sequence of SEQ ID NO:6 and 7, respectively;
   iv. Region comprising the amino acid at position 120 to the amino acid at position 205 of the LIC12615 protein as established by SEQ ID NO: 8, wherein the specific region selected consists of the amino acid sequence of SEQ ID NO:9;
   v. Region comprising amino acid position 70 to amino acid position 155 of protein LIC11636 as established by SEQ ID NO: 10, wherein the specific region selected consists of the amino acid sequence of SEQ ID NO:11;
   vi. Region comprising the amino acid from position 108 to the amino acid from position 138 of protein LIC11352 as set forth by SEQ ID NO: 12, and region comprising the amino acid from position 220 to the amino acid from position 272 of the LIC11352 protein as established by SEQ ID NO: 12, where the specific region selected consists of the amino acid sequence of SEQ ID NO: 13 and 14, respectively;
   vii. Region comprising the amino acid at position 84 to the amino acid at position 155 of the LIC11122 protein as established by SEQ ID NO: 15, wherein the specific region selected consists of the amino acid sequence of SEQ ID NO:16;
   viii. Region comprising amino acid position 30 to amino acid position 105 of protein LIC11089 as established by SEQ ID NO: 17, wherein the specific region selected consists of the amino acid sequence of SEQ ID NO: 18;
   ix. Region comprising amino acid position 161 to amino acid position 203 of protein LIC10713 as established by SEQ ID NO: 19, wherein the specific region selected consists of the amino acid sequence of SEQ ID NO: 20; and
   x. Region comprising the amino acid at position 374 to the amino acid at position 464 of the LIC13434 protein as established by SEQ ID NO: 21, wherein the specific region selected consists of the amino acid sequence of SEQ ID NO: 22.
2. Recombinant chimeric protein according to item 1, in which each said region is connected by a glycine linker.
3. Recombinant chimeric protein according to item 2, wherein said linker comprises the amino acid sequence as set forth by SEQ ID NO: 23.
4. Recombinant chimeric protein, according to any one of items 1 to 3, comprising six histidine amino acids at the beginning of the sequence of said recombinant chimeric protein.
5. Recombinant chimeric protein according to any one of items 1 to 4, wherein its molecular weight ranges from 91.73 to 100 KDa, preferably 94.65 KDa.
6. Recombinant chimeric protein according to any one of items 1 to 5, comprising the amino acid sequence as set forth by SEQ ID NO: 24.
7. Host cell comprising the chimeric gene consisting of the nucleotide sequence as established by SEQ ID NO: 25 or degenerates thereof encoding a protein consisting of the amino acid sequence as established by SEQ ID NO: 2, 4, 6, 7, 9, 11, 13, 14, 16, 18, 20, 22, or 24.
8. Host cell according to item 7, wherein the organism consists of *Escherichia coli, Pichia pastoris,* mammalian cells or insect cells, preferably *E. coli.*
9. Process of obtaining the chimeric protein as defined in any of items 1 to 6, comprising the following steps:
   a) Transforming the host cell or the *"cell free"* protein expression kit with a recombinant plasmid induced by isopropyl β-D-1-thiogalactopyranoside (IPTG) comprising the chimeric gene as defined in item 7; and
   b) Purifying the recombinant chimeric protein obtained by chromatography.
10. Process according to item 9, wherein in step "a", induction is carried out at a temperature ranging from 18 to 37 °C for 3 to 16 hours, wherein induction is preferably carried out at 18 °C for 16 hours.
11. Process, according to item 9 or 10, in which in step "a" a concentration of 0.01 to 1 mM of IPTG is used, preferably 1 mM, and the strain used is *E. coli* BL21 DE3, in which the induction is done after the culture reaches an optical density (OD600nm) of 0.6, in Luria-Bertani (LB) medium, in a volume ranging from 200 to 800 mL.
12. Leptospirosis serological diagnostic kit comprising the chimeric protein as defined in any of items 1 to 6.
13. Kit, according to item 12, further comprising a veterinary and/or pharmaceutically acceptable vehicle.
14. Kit, according to item 12 or 13, comprising a biosensor platform for *point of care* testing.
*15. An in vitro* method for the early serological diagnosis of leptospirosis which comprises the following steps:
   a) Immobilizing 100 to 500 ng/per well of the chimeric protein as defined in any of items 1 to 6 in an ELISA well plate;
   b) After blocking the ELISA wells with an inert protein, adding the serum of an individual suspected of having leptospirosis at a dilution of 1:50 to 1:400, and incubating preferably for 1 to 2 hours at a temperature of 37 °C;
   c) After washing off the unbound antibodies, adding a solution containing peroxidase-conjugated anti-human IgM and anti-human IgG secondary antibodies at a dilution of 1:5,000 to 1:10,000, and then adding a chromogenic substrate.
*16. In vitro* method, according to item 15, which determines a positive diagnosis by the concomitant detection of IgM and IgG antibodies against said chimeric protein.
17. Use of the chimeric protein as defined in any of items 1 to 6 for the early serological diagnosis of leptospirosis by detecting IgM and/or IgG type antibodies in serum samples from patients with suspected leptospirosis.
18. Use, according to item 17, in which it is for ELISA experiments or for immobilization in biosensors for rapid *point-of-care* tests.
19. Expression cassette comprising regions of the nucleotide sequences as established by SEQ ID NO: 26, 28, 30, 33, 35, 37, 40, 42, 44, and 46, or degenerate sequences thereof, which encode the peptides as established by SEQ ID NO: 1, 3, 5, 8, 10, 12, 15, 17, 19, and 21, operably linked to a heterologous promoter.
20. Expression cassette according to item 19, wherein said regions consist of the nucleotide sequences as established by SEQ ID NO: 27, 29, 31, 32, 34, 36, 38, 39, 41, 43, 45, and 47.
21. Chimeric gene consisting of the nucleotide sequence as set forth by SEQ ID NO: 25 or degenerate sequences thereof that encode a protein consisting of the amino acid sequence as set forth by SEQ ID NO: 2, 4, 6, 7, 9, 11, 13, 14, 16, 18, 20, 22, or 24.

Thus, the embodiments presented in the present invention do not limit the totality of possibilities, and it will be understood that various omissions, substitutions and alterations can be made by a skilled technician without departing from the scope of the present invention.

It is expressly provided that all combinations of the elements that perform the same function substantially in the same manner to achieve the same results are within the scope of the invention. Substitutions of elements from one described embodiment to another are also fully intended and contemplated.

Those skilled in the art will appreciate the knowledge presented here and will be able to reproduce the invention in the embodiments presented and in other variants covered by the scope of the claims.

## Claims

1. Recombinant chimeric protein, **characterized in that** it comprises specific regions of proteins proven to be conserved and present on the surface of *L*. interrogans. interrogans selected from the group consisting of LIC10123, LIC13059, LIC13050, LIC12615, LIC11636, LIC11352, LIC11122, LIC11089, LIC10713, and LIC13434, where the specific regions consist of:
(i) Region comprising the amino acid at position 132 to the amino acid at position 191 of the LIC10123 protein as established by SEQ ID NO: 1, wherein the specific region selected consists of the amino acid sequence of SEQ ID NO:2;
(ii) Region comprising the amino acid at position 76 to the amino acid at position 150 of the LIC13059 protein as established by SEQ ID NO: 3, where the specific region selected consists of the amino acid sequence of SEQ ID NO:4;
(iii) Region comprising amino acid position 101 to amino acid position 140 of the LIC13050 protein as set forth by SEQ ID NO: 5, and region comprising amino acid position 288 to amino acid position 324 of the LIC13050 protein as set forth by SEQ ID NO: 5, wherein the specific region selected consists of the amino acid sequence of SEQ ID NO: 6 and 7, respectively;
(iv) Region comprising amino acid position 120 to amino acid position 205 of the LIC12615 protein as set forth by SEQ ID NO: 8, wherein the specific region selected consists of the amino acid sequence of SEQ ID NO: 9;
(v) Region comprising amino acid position 70 to amino acid position 155 of protein LIC11636 as established by SEQ ID NO: 10, wherein the specific region selected consists of the amino acid sequence of SEQ ID NO:11;
(vi) Region comprising the amino acid from position 108 to the amino acid from position 138 of protein LIC11352 as set forth by SEQ ID NO: 12, and region comprising the amino acid from position 220 to the amino acid from position 272 of the LIC11352 protein as established by SEQ ID NO: 12, where the specific region selected consists of the amino acid sequence of SEQ ID NO: 13 and 14, respectively;
(vii) Region comprising the amino acid at position 84 to the amino acid at position 155 of the LIC11122 protein as established by SEQ ID NO: 15, wherein the specific region selected consists of the amino acid sequence of SEQ ID NO: 16;
(viii) Region comprising amino acid position 30 to amino acid position 105 of protein LIC11089 as established by SEQ ID NO: 17, wherein the specific region selected consists of the amino acid sequence of SEQ ID NO:18;
(ix) Region comprising amino acid position 161 to amino acid position 203 of protein LIC10713 as established by SEQ ID NO: 19, wherein the specific region selected consists of the amino acid sequence of SEQ ID NO: 20; and
(x) Region comprising the amino acid at position 374 to the amino acid at position 464 of the LIC13434 protein as established by SEQ ID NO: 21, wherein the specific region selected consists of the amino acid sequence of SEQ ID NO: 22.

2. Recombinant chimeric protein, according to claim 1, **characterized in that** each said region is connected by a glycine linker.

3. Recombinant chimeric protein, according to claim 2, **characterized in that** said linker consists of the amino acid sequence as established by SEQ ID NO: 23.

4. Recombinant chimeric protein, according to any one of claims 1 to 3, **characterized in that** it comprises six histidine amino acids at the beginning of the sequence of said recombinant chimeric protein.

5. Recombinant chimeric protein, according to any one of claims 1 to 4, **characterized in that** its molecular weight ranges from 91.73 to 100 KDa, preferably 94.65 KDa.

6. Recombinant chimeric protein, according to any one of claims 1 to 5, **characterized in that** it consists of the amino acid sequence as set forth by SEQ ID NO: 24.

7. Host cell, **characterized in that** it comprises the chimeric gene consisting of the nucleotide sequence as established by SEQ ID NO: 25 or degenerate sequences thereof that encode a protein consisting of the amino acid sequence as established by SEQ ID NO: 2, 4, 6, 7, 9, 11, 13, 14, 16, 18, 20, 22, or 24.

8. Host cell, according to claim 7, **characterized in that** the organism consists of *Escherichia coli, Pichia pastoris,* mammalian cells or insect cells, preferably *E. coli.*

9. Chimeric gene, **characterized in that** it consists of the nucleotide sequence as established by SEQ ID NO: 25 or degenerate sequences thereof that encode a protein consisting of the amino acid sequence as established by SEQ ID NO: 2, 4, 6, 7, 9, 11, 13, 14, 16, 18, 20, 22, or 24.

10. Process for obtaining the chimeric protein as defined in any one of claims 1 to 6, **characterized in that** it comprises the following steps:
a) Transforming the host cell or the cell-free protein expression kit with an isopropyl β-D-1-thiogalactopyranoside (IPTG)-induced recombinant plasmid comprising the chimeric gene as defined in claim 9; and
b) Purifying the recombinant chimeric protein obtained by chromatography.

11. Process, according to claim 10, **characterized in that**, in step "a", the induction is carried out at a temperature ranging from 18 to 37 °C for 3 to 16 hours, in which preferably the induction is carried out at 18 °C for 16 hours.

12. Process, according to claim 10, **characterized in that**, in step "a", the host cell is preferably selected from the group comprising *Escherichia coli, Pichia pastoris,* mammalian cells or insect cells, preferably *E. coli.*

13. Process, according to claim 10 or 11, **characterized in that**, in step "a", a concentration of 0.01 to 1 mM of IPTG is used, preferably 1 mM, and that the host cell used is *E. coli* BL21 DE3.

14. Process, according to claim 10 or 11 or 12 or 13, **characterized in that**, in step "a", the induction is carried out after the culture reaches an optical density (OD600nm) of 0.6, in Luria-Bertani (LB) medium, in a volume ranging from 200 to 800 mL.

15. Kit for serological diagnosis of leptospirosis, **characterized in that** it comprises the chimeric protein as defined in any one of claims 1 to 6.

16. Kit, according to claim 15, **characterized in that** it further comprises a veterinary and/or pharmaceutically acceptable vehicle.

17. Kit, according to claim 15 or 16, **characterized in that** it comprises a biosensor platform for *point of care* testing.

18. *In* vitro method for early serological diagnosis of leptospirosis, **characterized in that** it comprises the following stages:
a) Immobilizing 100 to 500 ng/per well of the chimeric protein as defined in any of claims 1 to 6 in an ELISA well plate;
b) After blocking the ELISA wells with a solution containing an inert protein, adding the serum of an individual with suspected leptospirosis at a dilution of 1:50 to 1:400, and incubating for 1 to 2 hours at 37 °C;
c) After washing off the unbound antibodies, adding a solution containing peroxidase-conjugated anti-human IgM and anti-human IgG secondary antibodies at a dilution of 1:5,00 to 1:10,000, and then adding a chromogenic substrate.

19. *In vitro* method, according to claim 18, **characterized in that** it determines a positive diagnosis by the concomitant detection of IgM and IgG antibodies against the chimeric protein.

20. Use of the chimeric protein as defined in any one of claims 1 to 6, **characterized in that** it is for the early serological diagnosis of leptospirosis by detecting IgM and/or IgG type antibodies in serum samples from patients with suspected leptospirosis.

21. Use according to claim 20, **characterized in that** it is for ELISA experiments or for immobilization in biosensors for rapid *point-of-care* tests.

22. Expression cassette, **characterized in that** it comprises regions of the nucleotide sequences as established by SEQ ID NO: 26, 28, 30, 33, 35, 37, 40, 42, 44, and 46, or degenerate sequences thereof, which encode the peptides as established by SEQ ID NO: 1, 3, 5, 8, 10, 12, 15, 17, 19, and 21, operably linked to a heterologous promoter.

23. Expression cassette, according to claim 22, **characterized in that** said regions consist of the nucleotide sequences as established by SEQ ID NO: 27, 29, 31, 32, 34, 36, 38, 39, 41, 43, 45, and 47.
